# EUROPEAN PATENT SPECIFICATION

(11) **EP 4 342 511 B1**
(45) Date of publication and mention of the grant of the patent: **22.10.2025**
(21) Application number: 22197306.8
(22) Date of filing: 23.09.2022
(51) Int. Cl.: A61M 15/00, A61B 5/00, A61M 16/00, G16H 10/60

(54) **INHALER SET**
INHALATOR-SET
ENSEMBLE INHALATEUR

(43) Date of publication of application: 27.03.2024
(73) Proprietor: Aptar Radolfzell GmbH, 78315 Radolfzell (DE)
(72) Inventor: Fabien, David, 29290 Saint Renan (FR)
(74) Representative: Witte, Weller und Partner Patentanwälte mbB Stuttgart

(56) References cited:
- WO-A1-2021/105440
- WO-A1-2022/171791
- AU-A1- 2015 318 726
- US-A1- 2013 269 685
- US-A1- 2020 360 630
- US-A1- 2021 146 070
- US-A1- 2022 273 891

## Description

### FIELD OF APPLICATION AND PRIOR ART

The invention relates to an inhaler set comprising an inhaler and a sensor unit, wherein said sensor unit is exchangeably attachable to the inhaler and is designed for detecting the use of the inhaler. Such inhaler sets are known from various prior art documents, e.g., from US 2017/0246406 A1, US 2016/0228657 A1, WO 2016/043601 A1, and US 2015/0100276 A1 as well as from US 2021/146070 A1 and WO 2022/171791 A1.

### OBJECT OF THE INVENTION

The object of the invention is to provide an inhaler set according to claim 1 allowing a reliable detection of the inhalation process without leading to a deterioration in inhalation comfort.

According to the invention an inhaler set is being provided comprising an inhaler and a sensor unit. The inhaler is designed as a powder inhaler. It has a housing with a mouthpiece forming the delivery opening for delivering an air flow containing a dose of said powder. Inside this housing the powder is stored prior to discharge. The dose of powder to be inhaled is atomized by the air flow caused by the user's inhalation and transported to the delivery opening.

The airflow for atomizing and transporting the powder is caused by the user's inhalation wherein a plurality of inlet openings is provided in the inhaler's housing though which air enters the inhalator and from where the air is being led past the prepared dose, atomizing said dose and delivering it to the delivery opening.

The sensor unit as second component of the inhaler set is provided for attachment to the inhaler. In the attached state the sensor unit covers at least one first air inlet opening such that the air entering the housing of the inhaler has to pass the sensor unit before entering the inhaler. Therefore, an inhalation sensor of the sensor unit is able to detect the air flow through said first air inlet opening. However, not all air inlet openings of the inhaler are covered in such a way by the sensor unit. At least one second air inlet opening is not covered by the sensor unit.

Therefore, the flow resistance that the air has to overcome passing through the inhaler and partially passing through the sensor unit is not much higher than when using the inhaler without the sensor unit. Nevertheless, the fraction of the air that enters through the at least one opening covered by the sensor unit, and which is therefore drawn in through the sensor unit, allows reliable detection and evaluation of the inhalation.

Preferably the inhaler provides a powder extraction location from which the powder is delivered to the mouthpiece by means of the airflow caused by the inhalation. At the extraction location a powder carrier surface can be provided on which the powder is being located. The air flow passing the extraction location is preferably passing through air passages provided in said surface and carrying the powder with it.

In particular, the extraction location can be different from the powder reservoir in which the powder is originally stored. A metering mechanism is preferably provided by means of which powder can be conveyed from the powder reservoir to an extraction location. Said metering mechanism provides a defined amount of powder from the reservoir and transports this amount to the extraction location. Preferably the metering mechanism has a conveying component which is movable relative to the powder reservoir between a filling position, in which powder is transferred from the powder reservoir onto the conveying component, and the extraction location. Preferably the powder carrier surface on which the powder is located for being carried to the mouthpiece by the air flow is part of the movable conveying component. The movable conveying component can in particular be a rotatable component which can be moved manually by the user using a rotatable actuator. Preferably, the conveying component is rotated unidirectionally with the rotatable actuator. The rotatable actuator is moved back and forth between two end positions, whereby it moves the conveying component in one direction of rotation and does not move the conveying component in the opposite direction of rotation, so that the conveying component is only moved stepwise in one direction of rotation.

The flow path of both the air from the first air inlet opening and the second air inlet opening can be led past the extraction location such that the powder is atomized by air coming from both air inlet openings, those covered by the sensor unit and those not being covered. It is however preferred that only the air from at least one first air inlet opening covered by the sensor unit is being used for atomizing the powder at the extraction location while air from at least one second air inlet opening not having passed through the sensor unit is being added to the air flow delivered at the mouthpiece downstream of the extraction location.

The sensor unit is attached to the inhaler such that the air entering the inhaler through the at least one first air inlet opening is covered by the sensor unit. The sensor unit can comprise an air inlet port being directly coupled to the first air opening port when attaching the sensor unit to the inhaler. However, in a preferred design the sensor unit defines a flow path together with the outer contour of the inhaler. Air entering the sensor unit through an air inlet port of the sensor unit is led into said flow path and from there enters the inhaler. Preferably an annular volume surrounding the inhaler portion inside the sensor unit is provided which is part of the flow path of the air through the sensor unit.

In the flow path between at least one air inlet port of the sensor and the at least one first air inlet opening of the inhaler a measuring channel is provided for measuring the air flow wherein an inhalation sensor measuring the air flowing through said measuring channel is provided. In a preferred embodiment a plurality of air inlet ports is provided on the sensor unit of which only one or some lead to the measuring channel while at least one other air inlet port is connected to the first air inlet opening of the installed inhaler bypassing the measuring channel. Even with a sensor unit providing only one air inlet port it can be advantageous to provide a bypass path in the flow path, along which the air passes from this single air inlet port of the sensor unit to the first air inlet opening of the inhaler, bypassing the measuring channel. Preferably the flow resistance through the measuring channel is higher than a flow resistance along the bypass path.

It has been found out that it is usually sufficient to measure only a small fraction of the air, namely only the part entering the inhaler through the sensor unit or even more only a part of this air flow through the sensor unit. This is not only sufficient to detect whether inhalation takes place but also to find out whether the inhalation is sufficiently powerful.

The sensor unit is adapted to the be attached to the inhaler. There are multiple possibilities for the design to achieve this. For example, the inhaler can have an attachment structure on its outer contour near its at least one first air inlet opening, and the sensor unit can be adapted to be coupled to this attachments structure.

However, in a preferred embodiment the sensor unit has a cup-shaped housing which defines an interior space into which the inhaler can be inserted from an open side with a bottom of the inhaler end opposite the delivery opening.

In order to isolate the space between the inhaler bottom and the inner contour of said sensor unit against the environment, it can be advantageous to provide on a circumferential side wall of the cup-shaped housing, an inwardly facing seal which after insertion of the inhaler into the sensor unit bears against an outer side of the inhaler housing. This seal can be made from a flexible material like rubber or TPE. The use of a cup-shaped sensor unit and the described seal leads to a sufficiently isolated flow path. Preferably the sensor unit is designed as an inseparable sensor unit which does not have to be disassembled for the purpose of inserting the inhaler. This can be achieved by said cup-shaped structure described above.

Preferably the seal is sufficiently flexible to allow the closure of the open side of the sensor unit by resting against an outer surface of the inhaler housing and by resting against an outer surface of a cap of the inhaler in case this cap is placed on the inhaler.

The bottom end of the inhaler which is being inserted into the cup-shaped housing of the sensor unit is preferably designed as or with an actuator rotatable against the housing or at least against the mouthpiece of the inhaler. This actuator is preferably provided for actuating the above-described metering device with which single doses of powder are being extracted from the powder reservoir.

In order to be able to handle this actuator while being covered by the sensor unit, the sensor unit, or a part of the sensor unit on the one hand side and the actuator of the inhaler on the other hand side are preferably designed with coupling geometries for rotationally secure coupling of the actuator and the sensor unit such as with ribs and grooves.

In particular it is preferred that on the side of the sensor unit the coupling geometry is provided on a carrier which is movable and preferably rotatable in a limited manner relatively to an outer housing of the sensor unit. Preferably the carrier and the outer housing are movable against each other by less than 30°, in particular preferably by less than 10°. Retaining members or retaining faces can be provided on the carrier and the housing for limiting the possible relative movement and providing two opposite end positions of said relative movement.

In order to prepare the inhaler for inhalation the user can grab the outer housing of the sensor unit and rotate it against the housing and/or the mouthpiece of the inhaler. At the beginning this leads to a movement of the whole sensor unit and of the actuator of the inhaler against the inhaler's housing and in particular against its mouthpiece. The joint movement ends as soon as the actuator reaches its end position. Continuation of the rotational movement leads to a relative movement between the outer housing of the sensor unit against the carrier which can not be moved further as it is coupled to the actuator in its end position relatively to the inhaler's housing.

The rotational movement of the carrier against the outer housing of the sensor unit can be used to detect that the user has rotated the inhaler's actuator against the mouthpiece and thus has prepared the inhaler for subsequent inhalation. This sensor will be described below.

The carrier of the sensor unit preferably also has a cup-shaped structure in order to receive the bottom end and in particular the actuator of the inhaler. In a preferred embodiment the carrier has a shape adapted to the bottom end of the inhaler such that after receiving the bottom end, said bottom end secured by friction or by interlocking elements in the carrier. According to a simple design this is realized by elastically deformable gripping surfaces which are provided on the carrier. When the inhaler is inserted into the sensor unit, these gripping surfaces are able to get in clamping contact with the bottom end of the inhaler, in particular with its actuator.

The sensor unit comprises electronic components for detecting the use of the inhaler. These components comprise some kind of energy source like a battery, a processor for processing data and at least one sensor for detecting the use of the inhaler, at least for detecting an air flow. In an embodiment of the sensor unit with the cup-shaped outer housing and the carrier being provided inside this outer housing, it is preferred that a receiving space is provided between the underside of the carrier or an intermediate wall beneath the carrier on the one hand side and the base of the outer housing on the other hand side. In this receiving space a control device comprising those electronic components is provided.

The inhalation sensor is advantageously a flow sensor which can be provided in said receiving space between the base of the housing and the carrier. The inhalation sensor can be designed as a thermal flow sensor. Such a thermal flow sensor comprises at least one heating element and at least on temperature sensor for sensing the temperature of the air caused by the heating element. In a preferred design the flow sensor comprises two temperature sensors and a heating element in between those two temperature sensors. This allows it to sense the temperature difference caused by heating element. Preferably the temperature sensors and the heating elements are realized in form of a prefabricated flow sensor module being located in the measuring channel or forming at least a part of said measuring channel. Said flow sensor module preferably has an inlet connector and an outlet connector, both of which preferably on the same side of the flow sensor module.

Besides the inhalation sensor at least one further sensor is preferably provided, in particular in said receiving space, for detection of the relative movement of the carrier and the sensor unit outer housing. Such a sensor is able to detect the relative movement and thus also the direction the carrier and the outer housing are moved relatively to each other.

The inhaler set preferably comprises a cap which when placed onto the inhaler housing covers and protects the mouthpiece. In particular, this cap can be a cap of the inhaler itself and thus being attachable to the inhaler even when the inhaler is not coupled to the sensor unit. Preferably the cup-shaped sensor unit is dimensioned such that the cap's lower end is surrounded by the sensor unit housing when the cap is in place. Another design in which the cap coupled to the sensor unit is also possible.

Preferably the sensor unit comprises a cap sensor for detecting the fitted cap. In preferred version of such a cap sensor a transmission lever is provided in an inner edge region of the cup-shaped housing. Said cap indirectly acts on the sensor when being moved and in particular tilted by the fitted cap. In particular a switch can be provided as a cap sensor for detecting the cap, said switch being switched by placing the cap on, wherein in particular the transmission lever acts on the switch for this purpose.

As written above, the sensor unit of the inhaler set preferably comprises actuation sensor for detecting a movement of an actuator of the inhaler thus detecting whether the inhaler is being prepared by the user by actuating the metering device of the inhaler.

This actuation sensor can be formed by a sensor which is designed to detect movement of a carrier of the sensor unit relatively to the housing of the outer sensor unit and/or to the control device which is usually fastened to the outer housing. Preferably this actuation sensor is designed such that it can detect both relative end positions between the carrier and the outer housing of the sensor unit.

Preferably a switch is provided as actuation sensor. This switch can by in particular a bistable switch having two switching positions depending on the relative position of the carrier and the outer housing. Alternatively, two switches can be provided, each of which being assigned to one switching position, a first switching position being achievable by a rotary movement of the carrier relative to the housing in a first direction of rotation, and a second switching position being achievable by a rotary movement of the carrier relative to the housing in a second direction of rotation.

The actuation sensor allows it to detect the preparation of an inhalation. This can be used to activate at least one further sensor in reaction, for example the inhalation sensor.

A further sensor which is preferably provided in the sensor unit of the inhaler set according to the invention is a coupling sensor for detecting the coupling of the sensor unit with an inhaler. On basis of this coupling sensor the control device is able to track the coupling and decoupling. Preferably the coupling sensor is designed as a switch being activated or deactivated by the coupling.

Preferably the coupling sensor is located in the region of the carrier into which the bottom of the inhaler is inserted during coupling. In particular, it is preferred to provide the coupling sensor itself under the carrier and thus between the base of the outer housing and the underside of the carrier. In order to detect the presence of the inhaler, an actuating portion can be provided which extends through an opening in a carrier of the sensor unit, said actuating portion being pressed down by the inhaler bottom and pressing on the switch. The actuating portion can be a segment of an intermediate housing wall between the base of the outer housing and the carrier, said portion being partially cut free and therefore movable relative to the surrounding parts of the intermediate housing wall.

A further sensor which can be advantageously provided on the sensor unit is an orientation sensor for detecting the orientation of the sensor unit and thus of the inhaler being coupled to the sensor unit. Depending on the type of inhaler it is important for a successful delivery of powder to hold the inhaler in the correct orientation during preparation and during inhalation. Usually during preparation, the inhaler has to be oriented vertically with the mouthpiece pointing upwards. During inhalation usually a horizontal orientation is correct. The orientation sensor is able to sense whether the inhaler is held correctly in the respective phases, in particular additionally using the inhaler sensor and/or the actuation sensor.

The sensors described above, namely the inhalation sensor/flow sensor, the cap sensor, the actuation sensor, the coupling sensor and the orientation sensor or some of these sensors can be part of the control device. The control device preferably has a circuit board arranged parallel to a bottom of the sensor unit. All or most of the sensors are preferably fixed directly on said circuit board.

The control device processes sensor data of the available sensors. Preferably it is adapted to activate and deactivate at least one sensor on basis of sensor data of other sensors. Although not limited, this refers primarily to the inhalation sensors which depending on its design consumes relatively much electrical energy. Thus, it is helpful to activate the inhalation sensor only when an inhalation will likely take place, in particular after a metering action has taken place which is being sensed by the actuation sensor and/or after the cap has been removed and the mouthpiece is thus accessible.

In order to show information and/or to give instructions to the user, the sensor unit has advantageously a display or at least one optical display element such as a LED. Preferably the sensor unit has a display or at least two optical elements and is thus capable of showing a directional information. Thus, the sensor unit can indicate in which direction of rotation the outer housing of the sensor unit must be moved relative to the housing of the inhaler in accordance with an inhalation preparation sequence.

Preferably the inhaler set does not consist of only the inhaler and the sensor unit but comprises also an external device or an application which runs on said external device like a smartphone. The external device and the control device are connectable to each other, preferably via a wireless connection on basis of Wi-Fi or Bluetooth. The sensor unit is therefore preferably equipped with a corresponding network interface. The application running on the external device is designed to receive data from the sensor unit. This can take place in order to store this data on the external device, in order to monitor the user's compliance and remind him of doses to be taken, when needed, and/or to send this data via the internet to a third party like a physician. Furthermore, the application running on the external device can analyze the usage of the inhaler set and give instructions to the user, for example regarding the general use of the device, the actuation, the correct orientation and/or the correct inhalation.

### BRIEF DESCRIPTION OF THE DRAWINGS

Further advantages and aspects of the invention are described and shown in the claims and in the following description of a preferred embodiment of the invention, which are explained below with reference to the figures.
Fig. 1 to 3 show an inhaler as known form the prior art.
Fig. 4 shows a sensor unit for coupling with the inhaler according to Fig. 1 to 3.
Fig. 5 and 6 show the inhaler and the sensor unit in coupled state.
Fig. 7 and 8 show the sensor unit from different perspectives.
Fig. 9 shows a sectional view of the sensor unit.
Fig. 10 shows an exploded view of the sensor unit, in particular of the carrier for coupling with the inhaler.
Fig. 11A to 12C show in Fig. 11A, 11B, 12A, 12B another sectional view of the sensor unit with a sectional plane just below the carrier and in fig. 11C and 12C a switching action caused by the movement of the carrier.
Fig. 13 shows the path of the air entering and passing through the sensor unit during inhalation.

### DETAILED DESCRIPTION OF THE EMBODIMENTS

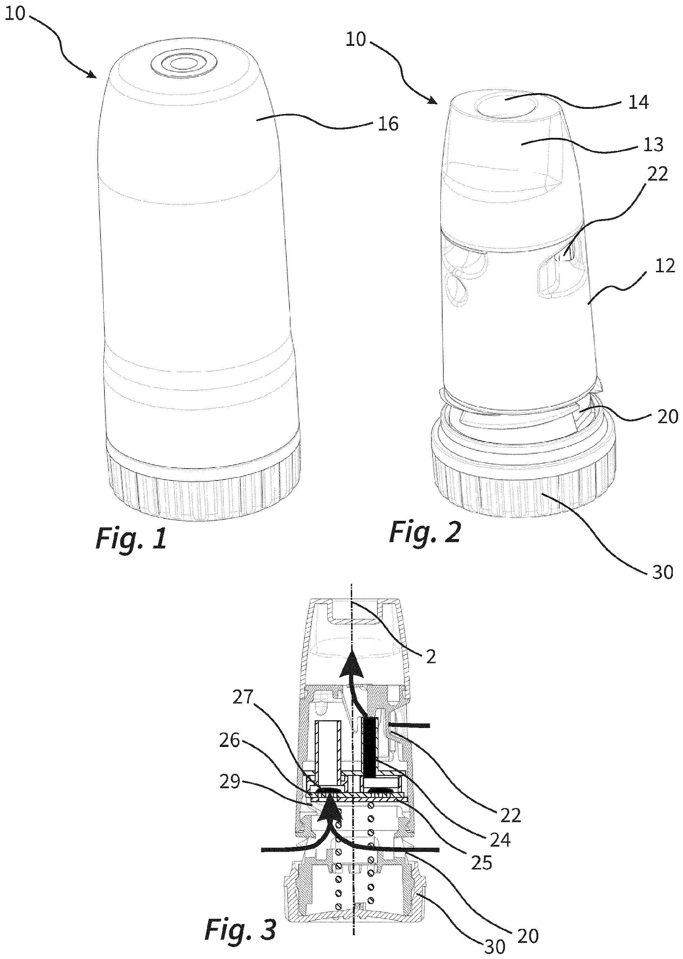

Fig. 1 to 3 show a conventional inhaler 10 which usually is called by its commercial name "Turbo-haler". The inhaler 10 is a powder inhaler for inhalation of a mixture of powder and air. The inhaler has a powder reservoir 24 inside its housing 12 from which a single dose can be isolated using a movable conveying component 26 for subsequent inhalation.

The housing 12 of the inhaler 10 has multiple air inlet openings 20, 22, namely lower inlet openings 20 and upper inlet openings 22. On one end of the housing 12 a mouthpiece 13 with a delivery opening 14 is provided. For inhalation the user takes the mouthpiece 13 in his mouth and inhales air. While doing so, air is entering the inhaler though the air inlet openings 20, 22. The air entering through the lower inlet openings 20 reaches the conveying component 26 onto which the powder 27 dose is placed, leading to the atomization of said powder dose and the delivering of this powder dose to the delivery opening 14 so that it is inhaled together with the air and thus reaching the upper or lower respiratory tract of the user. Downstream of the conveying component 26 the additional air entering through the upper inlet openings 22 is joining the stream originating from the lower inlet openings 20.

As can be seen from Fig. 1 the inhaler 10 has a cap 16 which can be placed over the mouthpiece 13 and the air inlet openings 20, 22 when the inhaler 10 is not in use.

Opposite to the mouthpiece 13 an actuator 30 is provided which is rotatable about the inhaler's main axis 2 in order to isolate a single dose of powder during preparation using the conveying component 26. In order to prepare a single dose, the actuator 30 is being rotated manually relative to the housing in a first direction. While doing so, the conveying component 26 is not rotated as the actuator 30 and the conveying component 26 are coupled to each other only in the opposite direction using a gripping latch 29 of the actuator 30.

Afterwards the actuator 30 is rotated in an opposite second direction together with the conveying component 26 so that powder 27 on the conveying component 26 reaches an extraction location on the left side in fig. 3 at which the powder dose is atomized by the air stream from the lower inlet openings 20 passing through an opening of an aperture plate 26 below the conveying component 26. At the same time a new dose of powder falls on the next segment of the rotatable conveying component 26 from the reservoir 24.

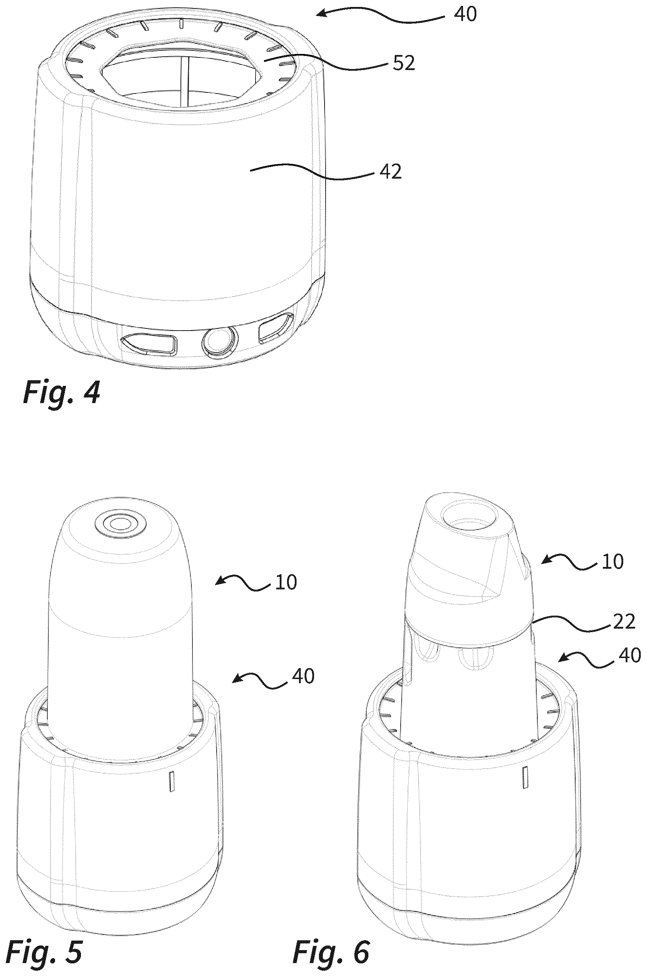

Fig. 4 shows a sensor unit 40 to be used with the inhaler 10. The sensor unit 40 has an outer housing 42 having a cup shape being open at the upper side for inserting the inhaler 10. At the inner side of the outer housing an inwardly facing seal 52 is located.

Fig. 5 and 6 show both the sensor unit 40 and the inhaler 10 being inserted in the sensor unit 40. In Fig. 5 the inhaler 10 is inserted together with its cap 16 with the lower edge of the cap 16 being inside the sensor unit 40. The seal 52 is sufficiently elastic to allow the cap 16 to be inserted this way while also being able to seal with the housing 12 of the inhaler 10 once the cap has been removed as shown in fig. 6. Preferably the seal is made of rubber or TPE.

With the inhaler 10 being inserted into the sensor unit 40 the lower air inlet openings 20 are placed inside the sensor unit 40 and thus isolated against the surrounding atmosphere by the seal 52 while the upper air inlet openings 22 are not covered by the sensor unit 40.

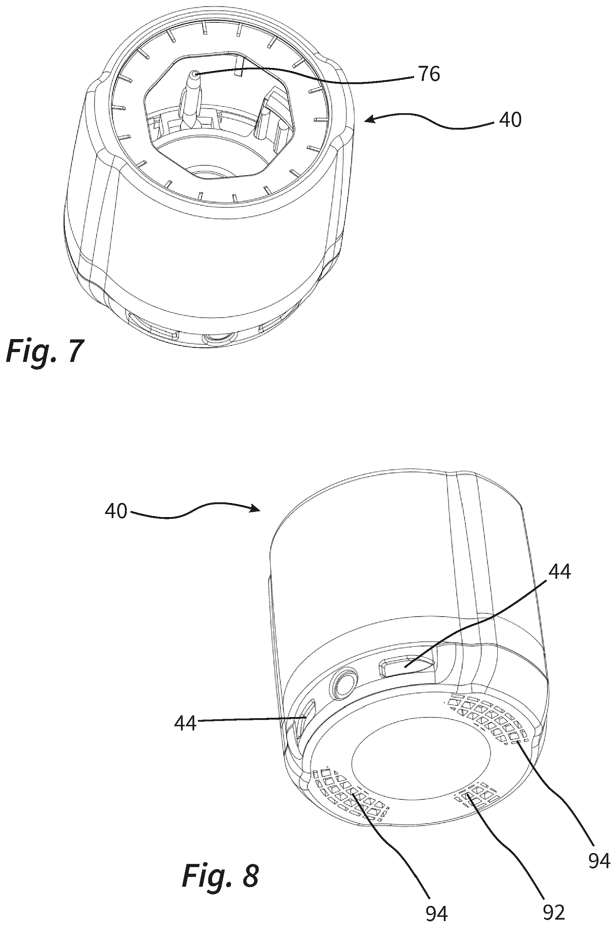

Fig. 7 and 8 show the sensor unit from different perspectives. From the perspective of Fig. 7 it can be seen that inside the sensor unit 40 an air outlet of an air channel 76 is located which will be described in detail below. From the perspective of Fig. 8 it can be seen that an on/off button and two arrow-shaped LED 44 are located on the outside of the sensor unit 40. Furthermore, it can be seen from fig. 8 that on the underside different air inlet ports 92, 94 are provided allowing air to enter the sensor unit 40.

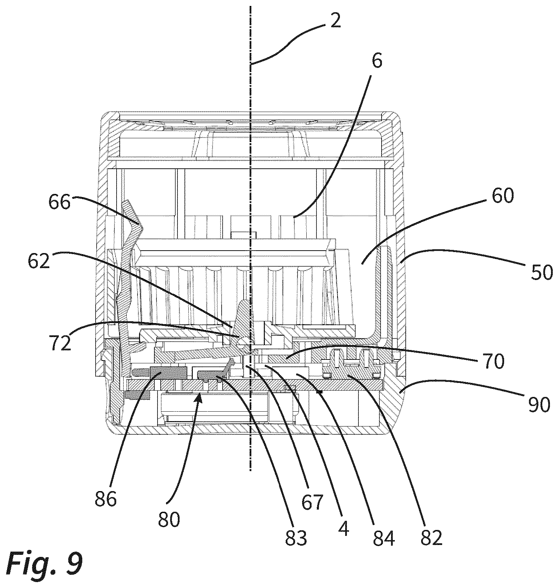

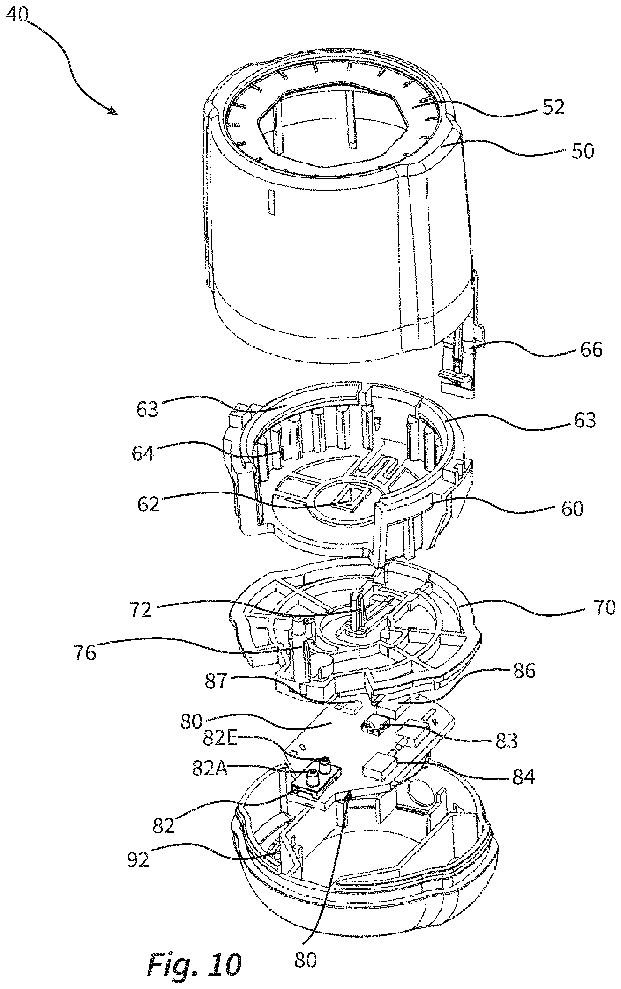

Fig. 9 shows the sensor unit 40 in a sectional view. Fig. 10 shows an explosion view on the sensor unit 40.

The outer housing 42 of the sensor unit 40 consists of two main parts, namely a housing base 90 and a roughly cylindrical sleeve-shaped upper part 50 fixed to said housing base 90. Together with an internal intermediate wall 70 the housing base 90 defines a receiving space 4 for accommodating an electronic control device 80 which comprises a processor and a wireless network interface for monitoring sensors of the sensor unit 40 and for transmitting data to an external device such as a smartphone. The control device 80 and its sensors 82, 83, 84, 86, 87 will be described in detail below.

The space 6 above the intermediate wall 70 laterally closed by the upper housing part 50 is the space into which the inhaler 10 is inserted. At the bottom of this space 6 a carrier 60 is provided which is rotatable in a limited manner against the rest of the sensor unit 40 about the main axis 2. Said carrier 60 is being provided for coupling with the rotatable actuator 30 of the inhaler 10. In order to allow a rotational movement of the actuator 30 by rotating the carrier 60 the carrier 60 is provided with a coupling geometry 64. In the example embodiment the coupling geometry comprises vertical ribs interacting with grooves on the outer circumferential face of the actuator 30. While the ribs of the coupling geometry are primarily intended for transferring torque from the carrier 60 to the actuator 30, additional gripping surfaces 63 of rubber or TPE or a comparable elastically deformable material are provided to prevent the inhaler form being pulled out accidentally out of the sensor unit 40.

The control device 80 has multiple sensors for detecting the usage of the inhaler 10. These sensors are described in the following:
The sensor unit 40 is able to detect the presence of an inserted inhaler 10. For this purpose, a coupling sensor 83 is provided in form of a switch in the center of the circuit board. The intermediate wall 70 is equipped with a deflectable actuation portion 72 having a pin pointing upwards through an opening 62 in the carrier 60. If the inhaler 10 is fully inserted into the sensor unit 40, it deflects the actuation portion 72 downwardly with its actuator 30, said deflection pressing the switch of the coupling sensor 83 and thus making the presence of the inhaler detectable for the control device 80.

The sensor unit 40 is furthermore able to detect whether the cap 16 is placed on the inhaler 10. For this purpose, a tiltable lever 66 is being provided in a side region of the space 6 defined by the upper housing part 50 and the intermediate wall 70. When the cap 16 is placed on the inhaler 10 as shown in fig. 5, its lower rim in located within the space 6, getting in contact with the lever 66 and tilting the upper part of the lever 66 outwardly. This makes the lower part of the lever 66 tilt inwardly and thus press on a switch 86 located onto the circuit board of the control device 80. This makes the presence of the cap 16 detectable for the control device 80.

Furthermore, an actuation sensor is provided for detecting the position of the carrier 60 relative to the outer housing 42 of the sensor unit 40.

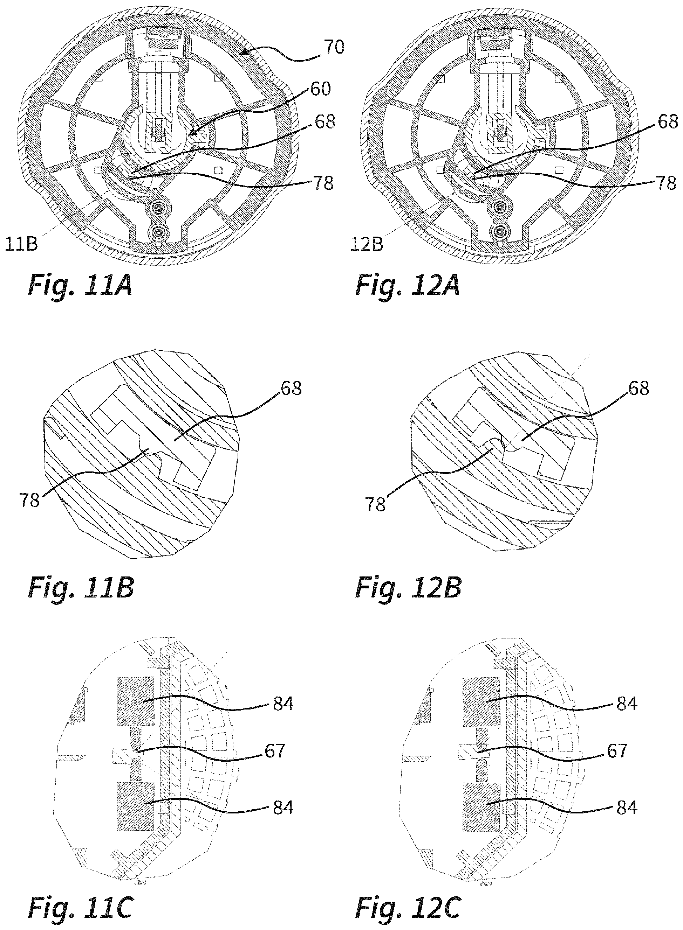

As shown in Fig. 11 the carrier 60 and the intermediate wall 70 are adapted to each other such that they can be snap together in two positions via a snapping mechanism 68, 78. When the user rotates the sensor unit 40 against the housing 12 of the inhaler 10, the carrier 60 and the outer housing 42 of the sensor unit 40 initially remain in an unchanged relative position until the carrier 60 together with the actuator 30 secured therein has reached an end position. This end position is defined by the limitation of the movability of the actuator 30 relative to the housing 12 of the inhaler 10. In this position powder is being transferred from the reservoir 24 to the conveying component 26 is the inhaler is in proper vertical orientation.

The continued manual application of torque causes the carrier 60 to move relative to the outer housing 42, pushing the snap mechanism 68, 78 from the first position of fig. 11A and 11B into its second position of fig. 12A and 12B.

When afterwards a rotation in opposite direction takes place to finish the metering process by bringing the conveying component 26 in its extraction location, again the outer housing 42 of the sensor unit 40 initially remains in an unchanged relative position until the carrier 60 together with the actuator 30 secured therein has reached the second end position relative to the housing 12 of the inhaler 10. Then again, the continued manual application of torque pushes the snap mechanism 68, 78 back into the first position.

In order to ensure that the rotational movement of the sensor unit's outer housing 42 and the carrier 60 takes place prior to the relative movement of the carrier 60 relatively to the outer housing 42, the torque for changing between the snap positions of the snapping mechanism 68, 78 is greater than the torque needed for rotating the actuator 30 relative to the housing 12 until the respective end position has been reached.

For sensing whether the snap mechanism 68, 78 is in its first or second position and thus for evaluating the preparation process, a system of two switches 84 is provided on the circuit board. Both switches 84 have a movable switching member, said switching members facing toward the respective other switch. In-between those switches a protrusion 67 on the underside of the carrier 60 is provided. If the snap mechanism 68, 78 is in its first position, one of the two switches 84 is being pressed in. If the snap mechanism is in its second position, the other one of the two switches 84 is being pressed in. This is shown in fig. 11C and 12C.

The fourth sensor of the sensor unit 40 is a flow sensor 82. This flow sensor is positioned and integrated such that during inhalation at least a part of the air stream from the air inlet port 92 of the sensor unit is flowing through the flow sensor 82 while air entering the sensor unit 40 through the other two air inlet ports 94 can directly flow to the air inlet opening 20.

The exact path of the air flowing through the flow sensor 82 is shown in Fig. 12 to 14.

As it can be seen from arrow 8A in Fig. 12 air entering through the air inlet port 92 reaches a first connector 82A of the flow sensor 82 where in enters the measuring channel 74.

The flow sensor 82 is designed as thermal flow sensor comprising two temperature sensors 82B, 82D and a heating element 82C. As shown by arrows 8B in Fig. 12 air flowing through the measuring channel 74 passes the first temperature sensor 82B, the heating element 82C and afterwards the second temperature sensor 82D. The temperature of the air in the measuring channel 74 is measured by the first temperature sensor 82B prior to the heating by the heating element 82C. Downstream of the heating element 82C the temperature of the air is measured once again by the second temperature sensor 82D. The more air flows through the measuring channel 74, the lower the temperature rise caused by the same heating power at the heating element 82C is. If no air flows through the measuring channel, the temperature measured by both temperature sensors 82B, 82D is roughly identical.

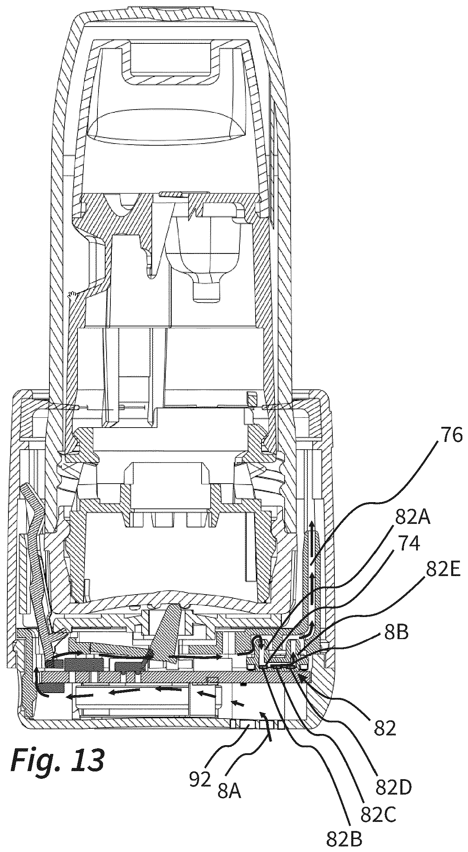

As shown in Fig. 13, the air exits through a second connector 82E of the flow sensor 82 into a subsequent air channel 76 being provided by the intermediate wall 70. Thus, the air is brought in the space between the inner side of the upper housing part 50 and the outer contour of the inhaler 10.

Only a small fraction of the air entering the inhaler 10 through the lower air inlet openings 20 passes through the measuring channel 74, while the main share of the air entering through the lower air inlet openings 20 enters the sensor unit 40 through the air inlet ports 94 and bypasses the measuring channel 74. It has however been found out, that this small fraction is sufficient for reliably estimating the total amount of air passing through the sensor unit 40 and entering the inhaler through the lower air inlet openings 20.

Furthermore, the control device comprises an orientation sensor 87 being able to measure the current orientation of the sensor unit 40 and thus of an inhaler 10 inserted into the sensor unit 40.

The control device is thus capable to sense whether an inhaler 10 is inserted into the sensor unit 40, whether the cap 16 has been removed, in which orientation the inhaler 10 is, in which preparation phase the inhaler 10 is and whether and how an inhalation has taken place.

This information from the sensors 82, 83, 84, 86, 87 is evaluated by the control device 80 and the information or information derived therefrom can be transmitted to an external device such as a smartphone via the wireless network interface, if necessary.

In particular, it can be provided that various sensors are only activated if certain states have been previously detected by other sensors. For example, some sensors, in particular the orientation sensor 87 and/or the flow sensor 82, are deactivated as long as no inhaler 10 is inserted into the sensor unit 40 or as long as its cap 16 is in place.

Since the flow sensor 82 has a relatively high-power consumption, it can also be useful to activate this flow sensor 82 only after it has been detected by means of the activation sensor 84, taking into account the orientation sensor 87, that a powder dose has been placed at the extraction location while the inhaler 10 has been in vertical orientation.

The control device 80 can further be used to provide assistance to the user via the LEDs, via a loudspeaker or via the external device. For example, after the cap 16 is removed, it can be checked using the orientation sensor 87 if the inhaler 10 is in a vertical orientation, and then indicate via the LEDs 44 which direction the actuator 30 needs to be turned. Once this process is complete by rotating the actuator in the first direction and afterwards in the opposite second direction, the conveying component 26 is provided with the powder dose 27 at the extraction location and the inhalation can begin. The inhalation can be supported, for example, by detecting the orientation and emitting a release sound when the inhaler is in a horizontal orientation or by signaling via both LED 44, for example by flashing green.

## Claims

1. Inhaler set having the following features:
a. the inhaler set has an inhaler (10) and a sensor unit (40), which can be exchangeably attached to the inhaler (10) for detecting the use of the inhaler (10), and
b. the inhaler (10) is designed as a powder inhaler and has a housing (12) with a delivery opening (14) designed as a mouthpiece and with a plurality of air inlet openings (20, 22), and
c. powder to be inhaled is stored inside the inhaler (10) prior to discharge, said powder being atomized and being transported to the delivery opening (14) by means of an air flow caused by the inhalation from the air inlet openings (20, 22) to the delivery opening (14), and
d. the sensor unit (40) is provided for attachment to the inhaler (10), wherein the sensor unit can be attached to the inhaler (10) in such a way that at least one first air inlet opening (20) is covered by the sensor unit (40) in the attached state such that the air is drawn in through the sensor unit (40) and at least one second air inlet opening (22) is not covered by the sensor unit (40) in the attached state, and
e. the sensor unit (40) comprises an inhalation sensor (82) for detecting the air flow through the first air inlet opening (20).

2. Inhaler set according to claim 1, having the following further features:
a. the inhaler (10) having a powder extraction location from which the powder is delivered to the mouthpiece by means of the airflow caused by the inhalation; and
b. a flow path from the first air inlet opening (20) to the delivery opening (14) passes through this extraction location; and
c. a flow path from the second air inlet opening (22) to the delivery opening (14) does not pass through said extraction location,
preferably with the following additional feature:
d. a surface provided with air passages is provided at the extraction location, through which air flows during inhalation, said air flowing from the first air inlet opening (20) to the delivery opening (14).

3. Inhaler set according to claim 1, having the following further feature:
a. the inhaler (10) has a powder reservoir and a metering mechanism by means of which powder can be conveyed from the powder reservoir to an extraction location,
preferably with the following additional feature:
b. the metering mechanism has a conveying component (26) which is movable relative to the powder reservoir, and which is movable between a filling position, in which powder passes from the powder reservoir (24) onto the conveying component (26), and the extraction location.

4. Inhaler set according to any of the preceding claims, having the following further features:
a. the sensor unit (40) together with the outer contour of the inhaler (10) defines a flow path from an air inlet port (92) of the sensor unit (40) to the first air inlet opening (20) of the inhaler (10), and
b. a measuring channel (74) is provided in the flow path, wherein the inhalation sensor (82) measures the air flowing through said measuring channel (74) for detecting the inhalation, and
c. a bypass path is provided in the flow path, along which the air passes from the air inlet port (92) of the sensor unit (40) to the first air inlet opening (22), bypassing the measuring channel (74),
preferably with the following additional feature:
d. a flow resistance through the measuring channel (74) is higher than a flow resistance along the bypass path.

5. Inhaler set according to any of the preceding claims, having the following additional feature:
a. the sensor unit (40) has a cup-shaped housing (42) which defines an interior space into which the inhaler (10) can be inserted from an open side with a bottom end opposite the delivery opening (14),
preferably with at least one of the following additional features:
b. the inhaler (10) has, at its bottom end a rotatable actuator (30) for actuating the metering device, the sensor unit (40) being designed with a coupling geometry (g) for rotationally secure coupling with the actuator (30), in particular the coupling geometry (64) being provided on a carrier (60) which can be moved with respect to the housing (42) of the sensor unit (40), and/or
c. the sensor unit (40) has, on a circumferential side wall of the cup-shaped housing, an inwardly facing seal (52) which after insertion of the inhaler (10) into the sensor unit (40) bears against an outer side of the inhaler (10), and/or
d. the sensor unit (40) is designed as an inseparable sensor unit (40) which does not have to be disassembled for the purpose of inserting the inhaler (10).

6. Inhaler set according to claim 5, having the following additional feature:
a. a carrier (60) is provided within the housing (42) of the sensor unit (40) for receiving the bottom end of the inhaler (10),
preferably having at least one of the following additional features:
b. the carrier (60) has a cup shape, and/or
c. elastically deformable gripping surfaces (63) are provided on the carrier (60) for clamping contact with the inhaler (10),
d. a receiving space for a control device (80) is provided in an intermediate space between the carrier (60) and a housing base (90), and/or
e. the carrier (60) is rotatable to a limited extent relative to the housing (42) of the sensor unit (40), at least one retaining element being provided which secures the carrier (60) and the housing (42) in two opposite end positions.

7. Inhaler set according to any of the preceding claims, having the following additional feature:
a. the inhalation sensor (82) is designed as a flow sensor,
preferably with the following additional feature:
b. the inhalation sensor (82) is designed as a thermal flow sensor and has two temperature sensors and a heating element arranged therebetween.

8. Inhaler set according to any of the preceding claims, having the following further features:
a. the inhaler (10) has a removable cap (16) which, when in place, covers the delivery opening (14), and
b. the sensor unit (40) comprises a cap sensor (86) for detecting the fitted cap (16),
preferably with at least one of the following additional features:
c. a transmission lever (66) is provided in an edge region of the cup-shaped housing (42), which is pivoted by putting on the cap (16), and/or
d. a switch (86) is provided as a cap sensor for detecting the cap (16), said switch being switched by placing the cap (16) on, wherein in particular the transmission lever (66) acts on the switch (86) for this purpose.

9. Inhaler set according to any of the preceding claims, having the following additional feature:
a. the sensor unit (40) comprises an actuation sensor (84) for detecting a movement of an actuator (30) of the inhaler (10), said actuator (30) being provided for actuating a metering device of the inhaler (10),
preferably with at least one of the following additional features:
b. the actuation sensor (84) for detecting the movement of the actuator (30) is formed by a sensor which is designed to detect movement of a carrier (60) of the sensor unit (40) with respect to the housing (42) of the sensor unit, and/or
c. the actuation sensor is formed by at least one switch, in particular by a bistable switch having at least two switching positions or by two switches, each of which is assigned to one switching position, a first switching position being achievable in particular preferably by a rotary movement of the carrier (60) relative to the housing (42) in a first direction of rotation, and a second switching position being achievable in particular preferably by a rotary movement of the carrier (60) relative to the housing (42) in a second direction of rotation.

10. Inhaler set according to any of the preceding claims, having the following additional feature:
a. the sensor unit (40) comprises a coupling sensor (83) for detecting the coupling of the sensor unit (40) with an inhaler (10),
preferably having at least one of the following additional features:
b. the coupling sensor (83) for detecting the coupling of the sensor unit with the inhaler is designed in the form of a switch, and/or
c. an actuating portion (72) is provided which preferably extends through an opening in a carrier (60) of the sensor unit (40), said actuating portion (72) pressing on the switch when the inhaler (10) is inserted.

11. Inhaler set according to any of the preceding claims, having the following additional feature:
a. the sensor unit (40) comprises an orientation sensor for detecting the orientation of the inhaler (10).

12. Inhaler set according to any of the preceding claims, having the following additional feature:
a. the sensor unit (40) comprises a control device (80), and
b. the control device (80) is adapted to activate and deactivate the inhalation sensor (82).

13. Inhaler set according to any of the preceding claims, having the following additional features:
a. the sensor unit (40) has a display and/or at least two optical display elements, and
b. the sensor unit (40) is designed to indicate, by means of the display or the display elements, in which direction of rotation a housing (42) of the sensor unit (40) must be moved relative to the housing (12) of the inhaler (10) in accordance with an inhalation preparation sequence.

14. Inhaler set according to claim 13 referring back to claim 8 or 9, having the following additional feature:
a. the control device (80) is adapted to activate or deactivate the inhalation sensor (82) in dependence on the cap sensor and/or the actuation sensor (84).

15. Inhaler set according to any of the preceding claims, having the following additional feature:
a. the inhaler set comprises a mobile device, in particular a smart phone, which is wirelessly connectable to the sensor unit (40).

## Patentansprüche

1. Inhalator-Satz mit folgenden Merkmalen:
a. der Inhalator-Satz weist einen Inhalator (10) und eine Sensoreinheit (40) auf, die lösbar am Inhalator (10) zum Erfassen der Benutzung des Inhalators (10) befestigbar ist, und
b. der Inhalator (10) ist als Pulverinhalator ausgebildet und weist ein Gehäuse (12) mit einer als Mundstück ausgebildeten Auslassöffnung (14) und mit einer Mehrzahl von Lufteinlassöffnungen (20, 22) auf, und
c. vor der Abgabe im Inhalator (10) gespeichertes Pulver wird mittels eines durch das Einatmen von den Lufteinlassöffnungen (20, 22) zur Auslassöffnung (14) verursachten Luftstroms zerstäubt und zur Auslassöffnung (14) transportiert, und
d. die Sensoreinheit (40) ist zum Anbringen am Inhalator (10) vorgesehen, wobei die Sensoreinheit am Inhalator (10) derart anbringbar ist, dass mindestens eine erste Lufteinlassöffnung (20) im angebrachten Zustand von der Sensoreinheit (40) abgedeckt ist, so dass die Luft durch die Sensoreinheit (40) angesaugt wird, und mindestens eine zweite Lufteinlassöffnung (22) im angebrachten Zustand nicht von der Sensoreinheit (40) abgedeckt ist, und
e. die Sensoreinheit (40) umfasst einen Inhalationssensor (82) zum Erfassen des Luftstroms durch die erste Lufteinlassöffnung (20).

2. Inhalator-Satz nach Anspruch 1 mit folgenden weiteren Merkmalen:
a. der Inhalator (10) weist eine Pulverentnahmestelle auf, von der aus das Pulver mittels des durch die Inhalation verursachten Luftstroms zum Mundstück gefördert wird; und
b. ein Strömungsweg von der ersten Lufteinlassöffnung (20) zur Auslassöffnung (14) verläuft durch diese Entnahmestelle; und
c. ein Strömungsweg von der zweiten Lufteinlassöffnung (22) zur Auslassöffnung (14) verläuft nicht durch diese Entnahmestelle,
vorzugsweise mit folgendem zusätzlichen Merkmal:
d. an der Entnahmestelle ist eine mit Luftdurchlässen versehene Oberfläche vorgesehen, durch die während der Inhalation Luft strömt, wobei die Luft von der ersten Lufteinlassöffnung (20) zur Auslassöffnung (14) strömt.

3. Inhalator-Satz nach Anspruch 1 mit folgendem weiteren Merkmal:
a. der Inhalator (10) weist ein Pulverreservoir und einen Dosiermechanismus auf, mittels dessen Pulver vom Pulverreservoir zu einer Entnahmestelle förderbar ist,
vorzugsweise mit folgendem zusätzlichen Merkmal:
b. der Dosiermechanismus weist ein Förderelement (26) auf, das relativ zum Pulverreservoir beweglich ist und zwischen einer Füllposition, in der Pulver vom Pulverreservoir (24) auf das Förderelement (26) gelangt, und der Entnahmestelle beweglich ist.

4. Inhalator-Satz nach einem der vorhergehenden Ansprüche mit folgenden weiteren Merkmalen:
a. die Sensoreinheit (40) definiert zusammen mit der Außenkontur des Inhalators (10) einen Strömungsweg von einer Lufteinlassöffnung (92) der Sensoreinheit (40) zur ersten Lufteinlassöffnung (20) des Inhalators (10), und
b. im Strömungsweg ist ein Messkanal (74) vorgesehen, wobei der Inhalationssensor (82) die durch den Messkanal (74) strömende Luft zur Erfassung der Inhalation misst, und
c. im Strömungsweg ist ein Bypassweg vorgesehen, entlang dessen die Luft von der Lufteinlassöffnung (92) der Sensoreinheit (40) zur ersten Lufteinlassöffnung (20) strömt, wobei der Messkanal (74) umgangen wird,
vorzugsweise mit folgendem zusätzlichen Merkmal:
d. ein Strömungswiderstand durch den Messkanal (74) ist größer als ein Strömungswiderstand entlang des Bypasswegs.

5. Inhalator-Satz nach einem der vorhergehenden Ansprüche mit folgendem zusätzlichen Merkmal:
a. die Sensoreinheit (40) weist ein topfförmiges Gehäuse (42) auf, das einen Innenraum definiert, in den der Inhalator (10) von einer offenen Seite mit einem der Auslassöffnung (14) gegenüberliegenden Bodenende einsetzbar ist,
vorzugsweise mit mindestens einem der folgenden zusätzlichen Merkmale:
b. der Inhalator (10) weist an seinem Bodenende einen drehbaren Aktuator (30) zum Betätigen der Dosier-einrichtung auf, wobei die Sensoreinheit (40) mit einer Kupplungsgeometrie (g) zur drehfesten Kupplung mit dem Aktuator (30) ausgebildet ist, wobei insbesondere die Kupplungsgeometrie (64) an einem Träger (60) vorgesehen ist, der relativ zum Gehäuse (42) der Sensoreinheit (40) bewegbar ist, und/oder
c. die Sensoreinheit (40) weist an einer Umfangsseitenwand des topfförmigen Gehäuses eine nach innen gerichtete Dichtung (52) auf, die nach dem Einsetzen des Inhalators (10) in die Sensoreinheit (40) gegen eine Außenseite des Inhalators (10) anliegt, und/oder
d. die Sensoreinheit (40) ist als untrennbare Sensoreinheit (40) ausgebildet, die zum Einsetzen des Inhalators (10) nicht zerlegt werden muss.

6. Inhalator-Satz nach Anspruch 5 mit folgendem zusätzlichen Merkmal:
a. innerhalb des Gehäuses (42) der Sensoreinheit (40) ist ein Träger (60) zum Aufnehmen des Bodenendes des Inhalators (10) vorgesehen,
vorzugsweise mit mindestens einem der folgenden zusätzlichen Merkmale:
b. der Träger (60) weist eine Topfform auf, und/oder
c. am Träger (60) sind elastisch verformbare Greifflächen (63) zum Klemmkontakt mit dem Inhalator (10) vorgesehen,
d. in einem Zwischenraum zwischen dem Träger (60) und einem Gehäuseboden (90) ist ein Aufnahmeraum für eine Steuervorrichtung (80) vorgesehen, und/oder
e. der Träger (60) ist relativ zum Gehäuse (42) der Sensoreinheit (40) in begrenztem Umfang drehbar, wobei mindestens ein Halteelement vorgesehen ist, das den Träger (60) und das Gehäuse (42) in zwei entgegengesetzten Endpositionen sichert.

7. Inhalator-Satz nach einem der vorhergehenden Ansprüche mit folgendem zusätzlichen Merkmal:
a. der Inhalationssensor (82) ist als Strömungssensor ausgebildet,
vorzugsweise mit folgendem zusätzlichen Merkmal:
b. der Inhalationssensor (82) ist als thermischer Strömungssensor ausgebildet und weist zwei Temperatursensoren und ein dazwischen angeordnetes Heizelement auf.

8. Inhalator-Satz nach einem der vorhergehenden Ansprüche mit folgenden weiteren Merkmalen:
a. der Inhalator (10) weist eine abnehmbare Kappe (16) auf, die in aufgesetztem Zustand die Auslassöffnung (14) abdeckt, und
b. die Sensoreinheit (40) umfasst einen Kappensensor (86) zum Erfassen der aufgesetzten Kappe (16),
vorzugsweise mit mindestens einem der folgenden zusätzlichen Merkmale:
c. in einem Randbereich des topfförmigen Gehäuses (42) ist ein Übertragungshebel (66) vorgesehen, der beim Aufsetzen der Kappe (16) verschwenkt wird, und/oder
d. ein Schalter (86) ist als Kappensensor zum Erfassen der Kappe (16) vorgesehen, wobei der Schalter durch Aufsetzen der Kappe (16) betätigt wird, wobei insbesondere der Übertragungshebel (66) hierzu auf den Schalter (86) einwirkt.

9. Inhalator-Satz nach einem der vorhergehenden Ansprüche mit folgendem zusätzlichen Merkmal:
a. die Sensoreinheit (40) umfasst einen Betätigungssensor (84) zum Erfassen einer Bewegung eines Aktuators (30) des Inhalators (10), wobei der Aktuator (30) zum Betätigen einer Dosier-einrichtung des Inhalators (10) vorgesehen ist,
vorzugsweise mit mindestens einem der folgenden zusätzlichen Merkmale:
b. der Betätigungssensor (84) zum Erfassen der Bewegung des Aktuators (30) ist durch einen Sensor gebildet, der zur Erfassung der Bewegung eines Trägers (60) der Sensoreinheit (40) relativ zum Gehäuse (42) der Sensoreinheit ausgebildet ist, und/oder
c. der Betätigungssensor ist durch mindestens einen Schalter gebildet, insbesondere durch einen bistabilen Schalter mit mindestens zwei Schaltstellungen oder durch zwei Schalter, von denen jeweils einer einer Schaltstellung zugeordnet ist, wobei eine erste Schaltstellung insbesondere vorzugsweise durch eine Drehbewegung des Trägers (60) relativ zum Gehäuse (42) in einer ersten Drehrichtung erreichbar ist und eine zweite Schaltstellung insbesondere vorzugsweise durch eine Drehbewegung des Trägers (60) relativ zum Gehäuse (42) in einer zweiten Drehrichtung erreichbar ist.

10. Inhalator-Satz nach einem der vorhergehenden Ansprüche mit folgendem zusätzlichen Merkmal:
a. die Sensoreinheit (40) umfasst einen Kupplungssensor (83) zum Erfassen der Kupplung der Sensoreinheit (40) mit einem Inhalator (10),
vorzugsweise mit mindestens einem der folgenden zusätzlichen Merkmale:
b. der Kupplungssensor (83) zum Erfassen der Kupplung der Sensoreinheit mit dem Inhalator ist in Form eines Schalters ausgebildet, und/oder
c. ein Betätigungsteil (72) ist vorgesehen, das vorzugsweise durch eine Öffnung in einem Träger (60) der Sensoreinheit (40) hindurchragt, wobei das Betätigungsteil (72) beim Einsetzen des Inhalators (10) auf den Schalter drückt.

11. Inhalator-Satz nach einem der vorhergehenden Ansprüche mit folgendem zusätzlichen Merkmal:
a. die Sensoreinheit (40) umfasst einen Orientierungssensor zum Erfassen der Orientierung des Inhalators (10).

12. Inhalator-Satz nach einem der vorhergehenden Ansprüche mit folgendem zusätzlichen Merkmal:
a. die Sensoreinheit (40) umfasst eine Steuervorrichtung (80), und
b. die Steuervorrichtung (80) ist eingerichtet, den Inhalationssensor (82) zu aktivieren und zu deaktivieren.

13. Inhalator-Satz nach einem der vorhergehenden Ansprüche mit folgenden zusätzlichen Merkmalen:
a. die Sensoreinheit (40) weist eine Anzeige und/oder mindestens zwei optische Anzeigeelemente auf, und
b. die Sensoreinheit (40) ist dazu eingerichtet, mittels der Anzeige oder der Anzeigeelemente anzuzeigen, in welche Drehrichtung ein Gehäuse (42) der Sensoreinheit (40) relativ zum Gehäuse (12) des Inhalators (10) entsprechend einer Inhalationsvorbereitungssequenz bewegt werden muss.

14. Inhalator-Satz nach Anspruch 13 in Rückbezug auf Anspruch 8 oder 9 mit folgendem zusätzlichen Merkmal:
a. die Steuervorrichtung (80) ist eingerichtet, den Inhalationssensor (82) abhängig vom Kappensensor und/oder vom Betätigungssensor (84) zu aktivieren oder zu deaktivieren.

15. Inhalator-Satz nach einem der vorhergehenden Ansprüche mit folgendem zusätzlichen Merkmal:
a. der Inhalator-Satz umfasst ein mobiles Gerät, insbesondere ein Smartphone, das drahtlos mit der Sensoreinheit (40) verbindbar ist.

## Revendications

1. Ensemble inhalateur présentant les caractéristiques suivantes :
a. l'ensemble inhalateur présente un inhalateur (10) et une unité de capteur (40), qui peuvent être fixés de manière interchangeable à l'inhalateur (10) pour détecter l'utilisation de l'inhalateur (10), et
b. l'inhalateur (10) est conçu en tant qu'inhalateur de poudre et présente un boîtier (12) doté d'une ouverture de distribution (14) conçue en tant qu'embout buccal et d'une pluralité d'ouvertures d'entrée d'air (20, 22), et
c. une poudre à inhaler est stockée à l'intérieur de l'inhalateur (10) avant d'être libérée, ladite poudre étant atomisée et étant transportée vers l'ouverture de distribution (14) au moyen d'un flux d'air provoqué par l'inhalation depuis les ouvertures d'entrée d'air (20, 22) vers l'ouverture de distribution (14), et
d. l'unité de capteur (40) est prévue pour être fixée à l'inhalateur (10), dans lequel l'unité de capteur peut être fixée à l'inhalateur (10) de telle sorte qu'au moins une première ouverture d'entrée d'air (20) soit recouverte par l'unité de capteur (40) à l'état fixé de telle sorte que l'air soit aspiré à travers l'unité de capteur (40) et qu'au moins une seconde ouverture d'entrée d'air (22) ne soit pas recouverte par l'unité de capteur (40) à l'état fixé, et
e. l'unité de capteur (40) comprend un capteur d'inhalation (82) permettant de détecter le flux d'air à travers la première ouverture d'entrée d'air (20).

2. Ensemble inhalateur selon la revendication 1, présentant les autres caractéristiques suivantes :
a. l'inhalateur (10) présentant un emplacement d'extraction de poudre à partir duquel la poudre est distribuée à l'embout buccal au moyen du flux d'air provoqué par l'inhalation ; et
b. un trajet d'écoulement de la première ouverture d'entrée d'air (20) à l'ouverture de distribution (14) traverse cet emplacement d'extraction ; et
c. un trajet d'écoulement entre la seconde ouverture d'entrée d'air (22) et l'ouverture de distribution (14) ne passe pas par ledit emplacement d'extraction,
préférablement avec la caractéristique additionnelle suivante :
d. une surface pourvue de passages d'air est prévue au niveau de l'emplacement d'extraction, à travers laquelle s'écoule l'air lors de l'inhalation, ledit air s'écoulant de la première ouverture d'entrée d'air (20) à l'ouverture de distribution (14).

3. Ensemble inhalateur selon la revendication 1, présentant l'autre caractéristique suivante :
a. l'inhalateur (10) présente un réservoir de poudre et un mécanisme de dosage au moyen duquel la poudre peut être transportée du réservoir de poudre vers un point d'extraction,
préférablement avec la caractéristique additionnelle suivante :
b. le mécanisme de dosage présente un composant de transport (26) qui est mobile par rapport au réservoir de poudre, et qui est mobile entre une position de remplissage, dans laquelle la poudre passe du réservoir de poudre (24) sur le composant de transport (26), et l'emplacement d'extraction.

4. Ensemble inhalateur selon l'une des revendications précédentes, présentant les autres caractéristiques suivantes :
a. l'unité de capteur (40) définit conjointement avec le contour externe de l'inhalateur (10) un trajet d'écoulement depuis un orifice d'entrée d'air (92) de l'unité de capteur (40) jusqu'à la première ouverture d'entrée d'air (20) de l'inhalateur (10), et
b. un canal de mesure (74) est prévu dans le trajet d'écoulement, le capteur d'inhalation (82) mesurant l'air circulant à travers ledit canal de mesure (74) pour détecter l'inhalation, et
c. un trajet de dérivation est prévu dans le trajet d'écoulement, le long duquel l'air passe de l'orifice d'entrée d'air (92) de l'unité de capteur (40) à la première ouverture d'entrée d'air (22), en contournant le canal de mesure (74),
préférablement avec la caractéristique additionnelle suivante :
d. une résistance à l'écoulement à travers le canal de mesure (74) est supérieure à une résistance à l'écoulement le long du trajet de dérivation.

5. Ensemble inhalateur selon l'une quelconque des revendications précédentes, présentant la caractéristique additionnelle suivante :
a. l'unité de capteur (40) présente un boîtier en forme de coupelle (42) qui définit un espace intérieur dans lequel l'inhalateur (10) peut être inséré à partir d'un côté ouvert avec une extrémité inférieure en regard de l'ouverture de distribution (14),
préférablement avec au moins l'une des caractéristiques additionnelles suivantes :
b. l'inhalateur (10) présente, à son extrémité inférieure, un actionneur rotatif (30) servant à actionner le dispositif de dosage, l'unité de capteur (40) étant conçue avec une géométrie d'accouplement (g) permettant un accouplement solidaire en rotation avec l'actionneur (30), en particulier la géométrie d'accouplement (64) étant prévue sur un support (60) qui peut être déplacé par rapport au boîtier (42) de l'unité de capteur (40), et/ou
c. l'unité de capteur (40) présente, sur une paroi latérale circonférentielle du boîtier en forme de coupelle, un joint orienté vers l'intérieur (52) qui, après insertion de l'inhalateur (10) dans l'unité de capteur (40), repose contre un côté externe de l'inhalateur (10), et/ou
d. l'unité de capteur (40) se présente sous la forme d'une unité de capteur (40) inséparable qui ne doit pas être démontée dans le but d'une insertion de l'inhalateur (10).

6. Ensemble inhalateur selon la revendication 5, présentant la caractéristique additionnelle suivante :
a. un support (60) est prévu à l'intérieur du boîtier (42) de l'unité de capteur (40) pour recevoir l'extrémité inférieure de l'inhalateur (10),
préférablement ayant au moins l'une des caractéristiques additionnelles suivantes :
b. le support (60) a une forme de coupelle, et/ou
c. des surfaces de préhension élastiquement déformables (63) sont prévues sur le support (60) pour un contact de serrage avec l'inhalateur (10),
d. un espace de réception pour un dispositif de commande (80) est prévu dans un espace intermédiaire entre le support (60) et une base de boîtier (90), et/ou
e. le support (60) peut tourner dans une mesure limitée par rapport au boîtier (42) de l'unité de capteur (40), au moins un élément de retenue étant prévu pour solidariser le support (60) et le boîtier (42) dans deux positions d'extrémités opposées.

7. Ensemble inhalateur selon l'une quelconque des revendications précédentes, présentant la caractéristique additionnelle suivante :
a. le capteur d'inhalation (82) est conçu en tant que capteur de débit,
préférablement avec la caractéristique additionnelle suivante :
b. le capteur d'inhalation (82) est conçu en tant que capteur de flux thermique et présente deux capteurs de température et un élément chauffant disposé entre ceux-ci.

8. Ensemble inhalateur selon l'une des revendications précédentes, présentant les autres caractéristiques suivantes :
a. l'inhalateur (10) présente un capuchon amovible (16) qui, lorsqu'il est en place, recouvre l'ouverture de distribution (14), et
b. l'unité de capteur (40) comprend un capteur de capuchon (86) permettant de détecter le capuchon (16) ajusté,
préférablement avec au moins l'une des caractéristiques additionnelles suivantes :
c. un levier de transmission (66) est prévu dans une région marginale du boîtier en forme de coupelle (42), lequel levier pivote par enfilage du capuchon (16), et/ou
d. un commutateur (86) est prévu en tant que capteur de capuchon pour détecter le capuchon (16), ledit commutateur étant commuté par le placement du capuchon (16), dans lequel en particulier le levier de transmission (66) agit sur le commutateur (86) à cette fin.

9. Ensemble inhalateur selon l'une quelconque des revendications précédentes, présentant la caractéristique additionnelle suivante :
a. l'unité de capteur (40) comprend un capteur d'actionnement (84) permettant de détecter un mouvement d'un actionneur (30) de l'inhalateur (10), ledit actionneur (30) étant prévu pour actionner un dispositif de dosage de l'inhalateur (10),
préférablement avec au moins l'une des caractéristiques additionnelles suivantes :
b. le capteur d'actionnement (84) permettant de détecter le mouvement de l'actionneur (30) est formé par un capteur qui est conçu pour détecter un mouvement d'un support (60) de l'unité de capteur (40) par rapport au boîtier (42) de l'unité de capteur, et/ou
c. le capteur d'actionnement est formé par au moins un commutateur, en particulier par un commutateur bistable présentant au moins deux positions de commutation ou par deux commutateurs dont chacun est associé à une position de commutation, une première position de commutation pouvant être obtenue en particulier de préférence par un mouvement de rotation du support (60) par rapport au boîtier (42) dans un premier sens de rotation, et une seconde position de commutation pouvant être obtenue en particulier de préférence par un mouvement de rotation du support (60) par rapport au boîtier (42) dans un second sens de rotation.

10. Ensemble inhalateur selon l'une quelconque des revendications précédentes, présentant la caractéristique additionnelle suivante :
a. l'unité de capteur (40) comprend un capteur d'accouplement (83) permettant de détecter l'accouplement de l'unité de capteur (40) avec un inhalateur (10),
préférablement ayant au moins l'une des caractéristiques additionnelles suivantes :
b. le capteur d'accouplement (83) permettant de détecter l'accouplement de l'unité de capteur avec l'inhalateur est conçu sous la forme d'un commutateur, et/ou
c. une partie d'actionnement (72) fournie s'étend de préférence à travers une ouverture ménagée dans un support (60) de l'unité de capteur (40), ladite partie d'actionnement (72) appuyant sur le commutateur lorsque l'inhalateur (10) est inséré.

11. Ensemble inhalateur selon l'une quelconque des revendications précédentes, présentant la caractéristique additionnelle suivante :
a. l'unité de capteur (40) comprend un capteur d'orientation permettant de détecter l'orientation de l'inhalateur (10).

12. Ensemble inhalateur selon l'une quelconque des revendications précédentes, présentant la caractéristique additionnelle suivante :
a. l'unité de capteur (40) comprend un dispositif de commande (80), et
b. le dispositif de commande (80) est conçu pour activer et désactiver le capteur d'inhalation (82).

13. Ensemble inhalateur selon l'une quelconque des revendications précédentes, présentant les caractéristiques additionnelles suivantes :
a. l'unité de capteur (40) présente un affichage et/ou au moins deux éléments d'affichage optiques, et
b. l'unité de capteur (40) est conçue pour indiquer, au moyen de l'affichage ou des éléments d'affichage, dans quel sens de rotation un boîtier (42) de l'unité de capteur (40) doit être déplacé par rapport au boîtier (12) de l'inhalateur (10) selon une séquence de préparation d'inhalation.

14. Ensemble inhalateur selon la revendication 13 se référant à la revendication 8 ou 9, présentant la caractéristique additionnelle suivante :
a. le dispositif de commande (80) est conçu pour activer ou désactiver le capteur d'inhalation (82) en fonction du capteur de capuchon et/ou du capteur d'actionnement (84).

15. Ensemble inhalateur selon l'une quelconque des revendications précédentes, présentant la caractéristique additionnelle suivante :
a. l'ensemble inhalateur comprend un dispositif mobile, en particulier un téléphone intelligent, qui peut être connecté sans fil à l'unité de capteur (40).
